(19) Europäisches Patentamt
European Patent Office
Office européen des brevets

(11) Veröffentlichungsnummer: **0 052 199**
**A1**

(12) **EUROPÄISCHE PATENTANMELDUNG**

(21) Anmeldenummer: **81107462.4**

(22) Anmeldetag: **19.09.81**

(51) Int. Cl.³: **C 07 D 403/06**
//C07D209/20

(30) Priorität: **15.11.80 DE 3043259**

(43) Veröffentlichungstag der Anmeldung:
**26.05.82 Patentblatt 82/21**

(84) *Benannte Vertragsstaaten:*
**BE CH DE FR GB LI NL**

(71) Anmelder: **Degussa Aktiengesellschaft**
**Weissfrauenstrasse 9**
**D-6000 Frankfurt am Main 1(DE)**

(72) Erfinder: **Kleemann, Axel, Dr. Dipl-Chem.**
**Greifenhagenstrasse 25**
**D-6450 Hanau 9(DE)**

(72) Erfinder: **Samson, Marc, Dr.**
**Grünaustrasse 1**
**D-6450 Hanau 9(DE)**

(54) **Verfahren zur Herstellung von Tryptophan-hydantoin.**

(57) Tryptophan-hydantoin wird in der Weise hergestellt, dass man
a) eine Verbindung der allgemeinen Formel

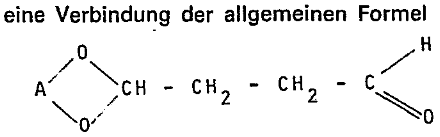

in der A eine unsubstituierte oder durch 1 bis 2 Methylgruppen substituierte Alkylengruppe mit 2 oder 3 Kohlenstoffatomen bedeutet, in wässriger oder wässrig-alkoholischer Lösung mit Cyanwasserstoff oder einer Cyanidionen liefernden Verbindung, Ammoniak oder einer Ammoniumionen liefernden Verbindung und Kohlendioxid oder einer Carbonationen liefernden Verbindung zur Reaktion bringt und
b) das erhaltene Reaktionsgemisch bei einem pH zwischen 0,1 und 4 mit Phenylhydrazin umsetzt.

**EP 0 052 199 A1**

Degussa Aktiengesellschaft
Weissfrauenstrasse 9, 6000 Frankfurt 1

Beschreibung:

Verfahren zur Herstellung von Tryptophan-hydantoin

Tryptophan ist eine essentielle Aminosäure, die häufig in Futtermitteln und Mischfuttern die limitierende Aminosäure darstellt. Da Tryptophan durch alkalische Hydrolyse von Tryptophan-hydantoin gewonnen werden kann, kommt dessen Synthese eine grosse Bedeutung zu.

Es sind bereits eine ganze Reihe von Verfahren zur Herstellung von Tryptophan-hydantoin bekannt, die letztlich entweder von Acrolein oder von Acrylnitril ausgehen und über mehrere Stufen verlaufen. Die bekannten Verfahren sind jedoch durchweg nicht voll befriedigend, weil sie entweder den Einsatz von schwer zugänglichen Reaktionsteilnehmern bzw. Hilfsstoffen erfordern oder in mindestens einer Reaktionsstufe nur verhältnismässig geringe Umsätze ergeben.

Gegenstand der Erfindung ist nun ein Verfahren zur Herstellung von Tryptophan-hydantoin, welches dadurch gekennzeichnet ist, dass man

a) eine Verbindung der allgemeinen Formel

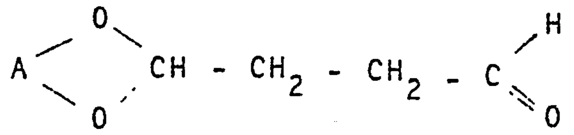 (I),

in der A eine unsubstituierte oder durch 1 bis 2 Methylgruppen substituierte Alkylengruppe mit 2 oder 3 Kohlenstoffatomen bedeutet, in wässriger oder wässrig-alkoholischer Lösung mit Cyanwasserstoff oder einer Cyanidionen liefernden Verbindung, Ammoniak oder einer Ammoniumionen liefernden Verbindung und Kohlendioxid oder einer Carbonationen liefernden Verbindung zur Reaktion bringt und

b) das erhaltene Reaktionsgemisch bei einem pH zwischen 0,1 und 4 mit Phenylhydrazin umsetzt.

Die beiden Reaktionsstufen a) und b) des erfindungsgemässen Verfahrens verlaufen mit hohen Umsätzen. Da auch die einzusetzenden Verbindungen der allgemeinen Formel (I) durch Hydroformylierung der entsprechenden 2-Vinyl-1,3-dioxolane bzw. 2-Vinyl-1,3-dioxane und letztere durch Acetalisierung von Acrolein mit den entsprechenden 1,2- bzw. 1,3-Glykolen leicht und in hohen Ausbeuten erhältlich sind, eröffnet das erfindungsgemässe Verfahren einen neuen, von Acrolein ausgehenden, vorteilhaften und kostengünstigen Weg zum Tryptophanhydantoin.

Beispiele für einzusetzende Verbindungen der allgemeinen Formel (I) sind 2-(2'-Formyläthyl)-1,3-dioxolan, 2-(2'-Formyläthyl)-4-methyl-1,3-dioxolan, 2-(2'-Formyläthyl)-4,5-dimethyl-1,3-dioxolan. 2-(2'-Formyläthyl)-1,3-dioxan,

BAD ORIGINAL

2-(2'-Formyläthyl)-4-methyl-1,3-dioxan oder 2-(2'-Formyläthyl)-5,5-dimethyl-1,3-dioxan.

Die Verbindungen der allgemeinen Formel (I) werden in einer ersten Reaktionsstufe in der für die Bildung von Hydantoinen aus Aldehyden an sich bekannten Weise mit Cyanwasserstoff oder einer Cyanidionen liefernden Verbindung, wie Natriumcyanid oder Kaliumcyanid, mit Ammoniak oder einer Ammoniumionen liefernden Verbindung, wie Ammoniumhydroxid oder Ammoniumchlorid, und mit Kohlendioxid oder einer Carbonationen liefernden Verbindung, wie Natrium- oder Kaliumbicarbonat, -carbonat oder -carbamat, umgesetzt. Es können auch Verbindungen eingesetzt werden, die gleichzeitig Cyanid- und Ammoniumionen liefern, wie Ammoniumcyanid, oder die gleichzeitig Ammonium- und Carbonationen liefern, wie Ammoniumcarbonat oder Ammoniumcarbamat.

Die Umsetzung in der ersten Reaktionsstufe erfolgt in Wasser oder in einem Gemisch aus Wasser und Methanol oder Äthanol. Sie kann in einem weiten Temperaturbereich vorgenommen werden. Bevorzugt wird eine Temperatur zwischen 30 und 90°C, weil in diesem Bereich eine zufriedenstellende Reaktionsgeschwindigkeit erreicht wird und der eventuell erforderliche Überdruck technisch kein Hindernis bildet.
Die Mengen der einzelnen Reaktionsteilnehmer können innerhalb weiter Grenzen variiert werden. Vorzugsweise werden je Mol Verbindung der allgemeinen Formel (I) 1 bis 1,5 Mol Cyanwasserstoff oder einer Cyanidionen liefernden Verbindung, 2 bis 15 Mol Ammoniak oder einer Ammoniumionen liefernden Verbindung und 1 bis 2 Mol Kohlendioxid oder einer Carbonationen liefernden Verbindung eingesetzt. Die Verbindungen der allgemeinen Formel (I) können gleichzeitig mit allen drei anderen

Reaktionsteilnehmern umgesetzt werden. Ebenso ist es aber auch möglich, sie zunächst nur mit der Cyanidkomponente und anschliessend mit den beiden anderen Komponenten gleichzeitig, oder zunächst nur mit der Cyanidkomponente, dann nur mit der Ammoniumkomponente und dann erst mit der Kohlendioxid- bzw. Carbonatkomponente umzusetzen. Besonders vorteilhaft ist es, wenn die Verbindung der allgemeinen Formel (I) in Methanol oder Äthanol gelöst und diese Lösung langsam zu einer auf die gewünschte Reaktionstemperatur erwärmten wässrigen Lösung oder Suspension der anderen Reaktionsteilnehmer zudosiert wird. Zur Erzielung eines hohen Umsatzes ist eine angemessene Nachreaktionszeit von beispielsweise etwa 5 Stunden nach dem Ende der Dosierung zu empfehlen.

Je nach den angewandten Reaktionsbedingungen enthält das Reaktionsgemisch nach Durchführung der ersten Reaktionsstufe neben dem zu erwartenden Hydantoin der allgemeinen Formel

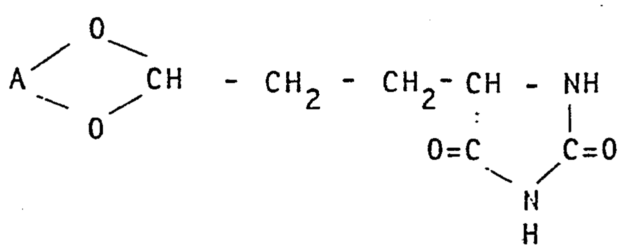

(II)

auch noch einen mehr oder weniger grossen Anteil an dem α-N-Carbamoyl-carbonsäureamid der allgemeinen Formel

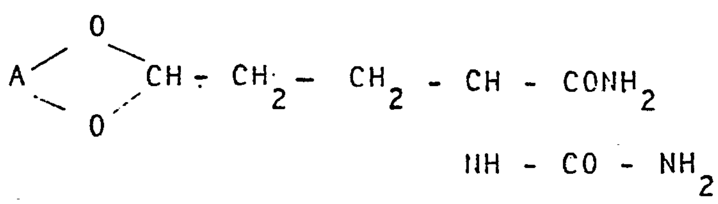

(III)

wobei in den Formeln (II) und (III) A wieder die oben bereits angegebene Bedeutung hat.

Eine Trennung der beiden Reaktionsprodukte ist jedoch nicht erforderlich, da sie beide bei der nachfolgenden Reaktionsstufe b) zu Tryptophan-hydantoin umgewandelt werden. Es kann aber zweckmässig sein, vor Durchführung der zweiten Reaktionsstufe die im rohen Reaktionsgemisch der ersten Reaktionsstufe enthaltenen Ammoniumsalze zu verkochen, den gegebenenfalls enthaltenen Alkohol abzudestillieren und das Reaktionsgemisch unter vermindertem Druck einzuengen.

In der Reaktionsstufe b) wird nun das in Stufe a) erhaltene Gemisch von Verbindungen der allgemeinen Formeln (II) und (III) bei einem pH zwischen 0,1 und 4 mit Phenylhydrazin direkt zu dem gewünschten Tryptophan-hydantoin umgesetzt. Der erforderliche pH zwischen 0,1 und 4, vorzugsweise zwischen 1 und 3, kann durch eine anorganische Säure, wie Schwefelsäure oder Phosphorsäure, durch eine organische Säure, wie Oxalsäure, Ameisensäure, Essigsäure, Benzolsulfonsäure oder p-Toluolsulfonsäure, oder durch einen stark sauren Ionenaustauscher eingestellt werden. Bevorzugt wird die Verwendung von Salzsäure. Da bei der Bildung des gewünschten Tryptophan-hydantoins aus den Verbindungen der allgemeinen Formel (II) ein und aus den Verbindungen der allgemeinen Formel (III) zwei Moleküle Ammoniak freigesetzt werden, ist es unter Umständen erforderlich, während der Reaktionsstufe b) durch Zugabe von Säure den pH-Wert nachzustellen. Auch in der zweiten Reaktionsstufe kann die Reaktionstemperatur innerhalb weiter Grenzen variiert werden. Zweckmässig sind Temperaturen zwischen 25 und 150 °C, vorzugsweise zwischen 70 und 120 °C.

Das Phenylhydrazin kann im Überschuss eingesetzt werden. Aus wirtschaftlichen Gründen ist es jedoch vorteilhafter, nur die der in der Reaktionsstufe a) eingesetzten Menge an der Verbindung der allgemeinen Formel (I) äquivalente Menge anzuwenden.

Das Phenylhydrazin kann mit dem aus der Reaktionsstufe a) erhaltenen Gemisch und der erforderlichen Menge an Säure vermengt und auf die gewünschte Reaktionstemperatur erhitzt werden. Ebenso ist es aber auch möglich, eine saure Lösung des Phenylhydrazins vorzulegen, zu erhitzen und das aus der Reaktionsstufe a) erhaltene Gemisch langsam zuzudosieren. In beiden Fällen ist eine Reaktionszeit von insgesamt etwa 3 bis 4 Stunden im allgemeinen ausreichend. Nach beendeter Reaktion kann die erhaltene Lösung des Tryptophan-hydantoins eingeengt und der Rückstand umkristallisiert werden. Man kann aber auch durch Abkühlen der Lösung das Tryptophan-hydantoin zur Kristallisation bringen und abtrennen. Die Reinheit des so erhaltenen Produkts liegt normalerweise über 95 %. Durch Umkristallisieren erhält man reines Tryptophan-hydantoin mit einem Schmelzpunkt von 213 bis 216°C.

Durch die nachfolgenden Beispiele soll die Erfindung näher verdeutlicht werden. Sofern nicht anders angegeben, bedeuten die Prozentangaben Gewichtsprozente.

Beispiel 1:

Zu einer Suspension aus 96 g Ammoniumcarbonat, 10,2 g Blausäure und 220 ml wässrigem Ammoniak (25 %ig) werden bei 35°C im Verlaufe einer Stunde 32,5 g 2-(2'-Formyl-äthyl)-1,3-dioxolan zugetropft und es wird fünf Stunden bei 40°C nachgerührt. Anschliessend werden durch Temperaturerhöhung (bis 100°C Kopftemperatur) die Salze verkocht. Die verbleibende wässrige Lösung wird mit konzentrierter Salzsäure auf pH 1 eingestellt und im Verlaufe einer Stunde langsam bei 90°C zu einer gerührten Lösung von 27 g Phenylhydrazin in 600 ml 0,1 N HCl zugetropft. Das Reaktionsgemisch wird anschliessend noch zwei Stunden lang auf der Temperatur von 90°C gehalten. Nach langsamem Abkühlen auf 10°C wird der gebildete Niederschlag abfiltriert und bei vermindertem Druck getrocknet. Die Ausbeute an Tryptophan-hydantoin beträgt 45,8 g (80 % der Theorie).

Elementaranalyse: $C_{12}H_{11}N_3O_2$

| | | | |
|---|---|---|---|
| Berechnet: | C 62,9 % | H 4,8 % | N 18,3 % |
| Gefunden: | C 61,6 % | H 4,9 % | N 18,2 %. |

Beispiel 2:

Zu einer Suspension aus 96 g Ammoniumcarbonat, 10,2 g Blausäure und 180 ml wässrigem Ammoniak (25 %ig) werden bei 40°C im Verlaufe einer Stunde 35,5 g 2-(2'-Formyl-äthyl)-4-methyl-1,3-dioxolan zugetropft und es wird vier Stunden bei 60°C nachgerührt. Nach dem Verkochen der

Salze bei 100°C wird die verbleibende wässrige Lösung mit konzentrierter Salzsäure auf pH 1,5 eingestellt und bei 95°C zu einer gerührten Lösung von 36,5 g Phenylhydrazin-hydrochlorid in 750 ml 0,1 N HCl zugegeben. Anschliessend wird noch zwei Stunden bei 95°C nachgerührt. Nach Abkühlen auf 10°C wird der gebildete Niederschlag abfiltriert und bei vermindertem Druck getrocknet. Die Ausbeute an Tryptophan-hydantoin beträgt 42,4 g (74 % der Theorie).

Beispiel 3:

Analog wie im Beispiel 1 werden 15,8 g 2-(2'-Formyläthyl-4,5-dimethyl-1,3-dioxolan mit 35 g Ammoniumcarbonat, 4,1 g Blausäure und 100 ml wässrigem Ammoniak (25 %ig) umgesetzt. Die so erhaltene wässrige Lösung wird mit 10,8 g Phenylhydrazin in 240 ml 0,1 N HCl bei 90°C gerührt. Die Ausbeute an Tryptophan-hydantoin beträgt 16,2 g (71 % der Theorie).

Beispiel 4:

Zu einer Suspension aus 83 g Ammoniumcarbonat, 11,7 g Blausäure und 250 ml wässrigem Ammoniak wird bei 50°C im Verlaufe einer Stunde eine Lösung von 41,4 g 2-(2'-Formyläthyl)-1,3-dioxan in 100 ml Methanol zugetropft und es wird noch drei Stunden lang bei 50°C nachgerührt. Anschliessend werden bis zu einer Kopftemperatur von 100°C das Methanol abdestilliert und die Salze verkocht. Die verbleibende wässrige Lösung

wird mit konzentrierter Salzsäure auf pH 1,2 eingestellt und bei 90°C im Verlaufe von 1,5 Stunden zu einer gerührten Lösung von 31 g Phenylhydrazin in 750 ml 0,1 N HCl zugetropft. Anschliessend wird das Reaktionsgemisch noch eine Stunde lang auf der Temperatur von 90°C gehalten. Nach dem Abkühlen auf 10°C wird der gebildete Niederschlag abfiltriert und bei vermindertem Druck getrocknet. Die Ausbeute an Tryptophan-hydantoin beträgt 48,8 g (74 % der Theorie).

Beispiel 5:

Analog wie im Beispiel 4 werden 45,5 g 2-(2'-Formyläthyl)-4-methyl-1,3-dioxan in Tryptophan-hydantoin umgewandelt. Die Ausbeute beträgt 52,7 g (80 % der Theorie).

Beispiel 6:

Zu einer Suspension aus 72 g Ammoniumcarbonat, 10,2 g Blausäure und 175 ml wässrigem Ammoniak (25 %ig) wird bei 35°C im Verlaufe einer Stunde eine Lösung von 43 g 2-(2'-Formyläthyl)-4,4-dimethyl-1,3-dioxan in 50 ml Methanol zugetropft und es wird noch vier Stunden lang bei 50°C nachgerührt. Anschliessend werden das Methanol abdestilliert und die Ammoniumsalze bei 100°C verkocht. Die verbleibende wässrige Lösung wird bei 80°C mit konzentrierter Salzsäure auf pH 1,5 eingestellt und mit dieser Temperatur im Verlaufe von zwei Stunden zu einer 90°C warmen gerührten Lösung von 27 g Phenylhydrazin in 600 ml 0,1 N HCl zugetropft. Anschliessend

wird noch eine Stunde lang bei 95°C nachgerührt und langsam auf 10°C abgekühlt. Der gebildete Niederschlag wird abfiltriert und bei vermindertem Druck getrocknet. Die Ausbeute an Tryptophan-hydantoin beträgt 45,3 g (79 % der Theorie).

Beispiel 7:

Zu einer Suspension aus 48 g Ammoniumcarbonat, 5,1 g Blausäure und 120 ml wässrigem Ammoniak (25 %ig) wird bei 35°C eine Lösung von 16,3 g 2-(2'-Formyläthyl)-1,3-dioxolan in 100 ml Äthanol zugetropft und es wird fünf Stunden lang bei 60°C nachgerührt. Die so erhaltene Lösung wird mit konzentrierter Salzsäure auf pH 1 eingestellt und es wird bei Raumtemperatur eine Lösung von 13,5 g Phenylhydrazin in 300 ml 0,1 N HCl zugegeben. Das Reaktionsgemisch wird zwei Stunden lang auf 90°C erhitzt. abgekühlt und bei vermindertem Druck eingeengt. Der Rückstand wird aus einer Mischung Wasser/Äthanol (50 : 50) umkristallsiert. Die Ausbeute an Tryptophan-hydantoin beträgt 19,7 g (68,5 % der Theorie).

18. September 1981
PAT/Dr.Sib-El

Degussa Aktiengesellschaft
Weissfrauenstrasse 9, 6000 Frankfurt 1

Verfahren zur Herstellung von Tryptophan-hydantoin

Patentansprüche:

1. Verfahren zur Herstellung von Tryptophan-hydantoin, dadurch gekennzeichnet, dass man

a) eine Verbindung der allgemeinen Formel

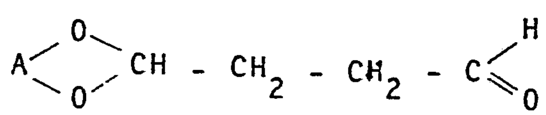

(I),

in der A eine unsubstituierte oder durch 1 bis 2 Methylgruppen substituierte Alkylengruppe mit 2 oder 3 Kohlenstoffatomen bedeutet, in wässriger oder wässrig-alkoholischer Lösung mit Cyanwasserstoff oder einer Cyanidionen liefernden Verbindung, Ammoniak oder einer Ammoniumionen liefernden Verbindung und Kohlendioxid oder einer Carbonationen liefernden Verbindung zur Reaktion bringt und

b) das erhaltene Reaktionsgemisch bei einem pH zwischen 0,1 und 4 mit Phenylhydrazin umsetzt.

2. Verfahren nach Anspruch 1, dadurch gekennzeichnet, dass man in der Reaktionsstufe a) den Cyanwasserstoff oder die Cyanidionen liefernde Verbindung in einer Menge zwischen 1 und 1,5 Mol, den Ammoniak oder die Ammoniumionen liefernde Verbindung in einer Menge zwischen 2 und 15 Mol und das Kohlendioxid oder die Carbonationen liefernde Verbindung in einer Menge zwischen 1 und 2 Mol, jeweils pro Mol eingesetzter Verbindung der allgemeinen Formel (I), einsetzt.

3. Verfahren nach Anspruch 1 oder 2, dadurch gekennzeichnet, dass man die Reaktionsstufe a) bei einer Temperatur zwischen 30 und 90°C durchführt.

4. Verfahren nach einem der Ansprüche 1 bis 3, dadurch gekennzeichnet, dass man die Reaktionsstufe b) bei einer Temperatur zwischen 25 und 150°C durchführt.

0052199

Europäisches Patentamt

# EUROPÄISCHER RECHERCHENBERICHT

Nummer der Anmeldung

EP 81 10 7462.4

| EINSCHLÄGIGE DOKUMENTE | | | KLASSIFIKATION DER ANMELDUNG (Int. Cl.3) |
|---|---|---|---|
| Kategorie | Kennzeichnung des Dokuments mit Angabe, soweit erforderlich, der maßgeblichen Teile | betrifft Anspruch | |
| Y | Chemical Abstracs, Band 70, Nr. 19, 12. Mai 1969, Columbus, Ohio, USA I. MAEDA et al. "Synthesis of DL-tryptophan from acrolein" Seite 395, Spalte 2 bis Seite 396, Spalte 1, Abstract Nr. 88224e & Bull. Chem. Soc. Jap., Band 41, Nr. 12, 1968, Seiten 2975 bis 2978 -- | 1 | C 07 D 403/06 //C 07 D 209/20 |
| Y | US - A - 3 419 551 (AJINOMOTO CO) * Spalte 4, Zeilen 49 bis 53 * -- | 1 | RECHERCHIERTE SACHGEBIETE (Int. Cl.3) |
| P,A | Chemical Abstracts, Band 94, Nr. 23, 8. Juni 1981, Columbus, Ohio, USA Seite 663, Spalte 2, Abstract Nr. 192335y | | C 07 D 209/20 C 07 D 403/06 |
| A | & JP - A - 80 136 279, 23. Oktober 1980 -- | | |
| A | Chemical Abstracts, Band 57, Nr. 4, 20. August 1962, Columbus, Ohio, USA J.N. COKER et al. "Synthesis and resolution of tryptophan" Spalte 4751de & J.Org. Chem., Band 27, 1962, Seiten 850 bis 854 ---- | | KATEGORIE DER GENANNTEN DOKUMENTE |

KATEGORIE DER GENANNTEN DOKUMENTE
X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 03-02-1982 | FROELICH |